# DEMANDE DE BREVET EUROPEEN

(11) **EP 0 537 060 A1**
(43) Date de publication de la demande: **14.04.1993**
(21) Numéro de dépôt: 92402713.9
(22) Date de dépôt: 05.10.1992
(51) Int. Cl.: A61B 19/00

(54) **Vis à os percutanée, destinée à supporter notamment un cadre de stéréotaxie**

(30) Priorité: 11.10.1991 FR 9112571
(71) Demandeur: SOCIETE DE FABRICATION DE MATERIEL ORTHOPEDIQUE SOFAMOR, F-75016 Paris (FR)
(72) Inventeur: Leriche, Bertrand, F-75014 Paris (FR)
(74) Mandataire: Martin, Jean-Paul

(57) **Abrégé**

La vis (1) comprend une extrémité formée par un téton (2) présentant une surface transversale plane (3) et une surface latérale cylindrique (4) raccordée à la surface plane (3) par un congé annulaire arrondi (5); elle comprend également une partie filetée (6) contigüe au téton (2) dans laquelle est ménagée une zone autotaraudeuse (8) qui se termine à la base du téton (2), une partie lisse cylindrique (9) étant de plus agencée entre sa partie filetée (6) et sa tête (11). La partie lisse (9) est raccordée à la tête (11) par une zone plane inclinée (12). Le téton arrondi (2) permet de refouler sans l'abimer toute muqueuse ou aponévrose située derrière un os traversé par la vis. Les parties lisses (9 et 12) évitent d'endommager la peau du patient. Cette vis est destinée à être mise en place de façon très précise et à demeurer ancrée dans le patient pendant toute la durée nécessaire aux investigations prévues, ce qui évite des perforations répétées du crâne ou de tout os à chaque examen.

## Description

La présente invention a pour objet une vis à os percutanée, destinée à supporter notamment un cadre de stéréotaxie permettant des investigations dans la boîte crânienne d'un patient.

On sait que certaines techniques médicales d'investigations ou de traitements d'infections intracérébrales imposent la reposition exacte, millimétrique, du cerveau dans l'espace. On connaît ainsi des techniques diverses et complémentaires d'explorations radiologiques telles que l'angiographie, le scanner et l'exploration en résonance magnétique nucléaire. L'intérêt de ces explorations est de pouvoir être superposées lors de leur exploitation afin d'en tirer un maximum de données, sachant qu'elles sont irréalisables conjointement, pour raisons techniques :
- L'angiographie nécessite un matériel radiologique particulier et est effectuée sous anesthésie, souvent générale.
- Le scanner nécessite une enceinte spécifique.
- L'IRM (Imagerie en résonance magnétique) impose une structure encore plus spécifique du fait de l'importance des champs magnétiques.

Une reposition millimétrique du cerveau dans l'espace est également nécessaire pour la mise en oeuvre de certaines techniques thérapeutiques, soit de chirurgie en conditions stéréotaxiques, soit d'irradiations répétées pour des affections tumorales ou des malformations vasculaires (angiomes).

La mise en place d'un cadre de référence, métallique ou non, fixé sur la boîte crânienne permet la réalisation topographiquement superposable d'investigations : artériographie, ventriculographie, scanner, IRM, et la réalisation de traitements, ce qui constitue le principe de la stéréotaxie. Mais cela suppose un repositionnement à chaque fois du cadre, et donc de la fixation de l'ancrage crânien.

Deux modalités techniques sont à cet effet possibles :
- Soit un repérage refait à chaque fois, utilisant le calcul par coordonnées tridimensionnelles. Ce calcul est actuellement relativement aisé, mais la précision de repose n'est pas toujours millimétrique, compte tenu de la difficulté de repositionnement des pointeaux du cadre dans les trous osseux.
   Il en résulte pour le patient des désagréments évidents : sa boîte crânienne doit en effet subir des perforations, des incisions du cuir chevelu et des anesthésies locales répétées à chaque repose, engendrant des risques d'infection et une gêne douloureuse. Pour le chirurgien la répétition de ces opérations de pose nécessite un temps considérable, à la recherche d'un réglage précis.
- Soit le repositionnement exact, millimétrique, du cadre dans la structure osseuse de la boîte crânienne par l'intermédiaire de vis à os percutanées.

Cette dernière technique, certainement la plus précise, implique la repose itérative du cadre sur la boîte crânienne, sur des vis percutanées (c'est-à-dire dont la tête dépasse à l'extérieur de la peau du patient) ancrées dans la structure osseuse, plusieurs jours de suite ou éventuellement plusieurs jours par semaine durant plusieurs semaines, lors d'irradiations séquentielles.

L'invention a donc pour but de proposer une solution satisfaisante à ce problème, en réalisant une vis à os percutanée pouvant rester en place dans la boîte crânienne du patient après un réglage initial précis, et ce pendant plusieurs semaines pour investigation ou thérapeutique avec une bonne tolérance cutanée et osseuse, et une facilité de repositionnement itératif du cadre.

Conformément à l'invention, la vis à os percutanée comporte une extrémité formée par un téton arrondi et émoussé, et dans sa partie filetée, contiguë au téton, est ménagée une zone autotaraudeuse constituée d'au moins une goujure formée par un plat bordé par des arêtes vives, et qui se termine au voisinage de la base du téton.

La conformation arrondie de l'extrémité de la vis évite, lors de l'ancrage par exemple crânien de la vis, tout risque d'endommagement de l'aponévrose ou muqueuse sous-jacente telle la dure-mère qui enveloppe le cerveau. Cette membrane est en effet légèrement repoussée par le téton arrondi de la vis, écartant tout risque d'oedème qui pourrait être provoqué par une arête coupante et limitant une éventuelle réaction inflammatoire ou hémorragique.

Le fait que la vis soit pourvue d'une partie autotaraudeuse évite de devoir aléser et tarauder le trou osseux.

Suivant une forme d'exécution possible de l'invention, le téton présente une surface transversale plane et une surface latérale cylindrique, raccordée à la surface plane par un congé annulaire arrondi.

La vis comprend avantageusement une partie lisse cylindrique, agencée entre sa partie filetée et une tête adaptée pour recevoir un outil de manoeuvre. Cette partie lisse est transcutanée lorsque la vis est en place, ménageant la trophicité de la peau.

D'autres caractéristiques et avantages de l'invention apparaîtront au cours de la description qui va suivre, faite en référence aux dessins annexés qui en illustrent une forme de réalisation à titre d'exemple non limitatif.

La figure 1 est une vue en coupe axiale à échelle très agrandie, d'une forme de réalisation de la vis à os percutanée selon l'invention.

La figure 2 est une vue en élévation longitudinale à la même échelle que la Fig.1, de la vis selon l'invention.

La figure 3 est une vue en perspective à la même échelle que les Fig.1 et 2, de la vis à os percutanée selon l'invention.

La figure 4 est une vue en élévation frontale de la tête de la vis suivant la direction F de la Fig.3.

La figure 5 est une vue schématique en élévation de la tête d'un patient sur laquelle est posé un cadre de stéréotaxie, ce cadre étant supporté par des vis crâniennes conformes à l'invention.

La figure 6 est une vue de dessus correspondant à la Fig.5, montrant la mise en place du cadre.

La figure 7 est une vue en élévation partielle du cadre et d'une des vis du dispositif des Fig.5 et 6.

La figure 8 est une vue mi-coupe partielle mi-élévation de la paroi d'une boîte crânienne et d'une vis ancrée dans celle-ci, prête à recevoir un cadre tel que celui des Fig.5 et 6.

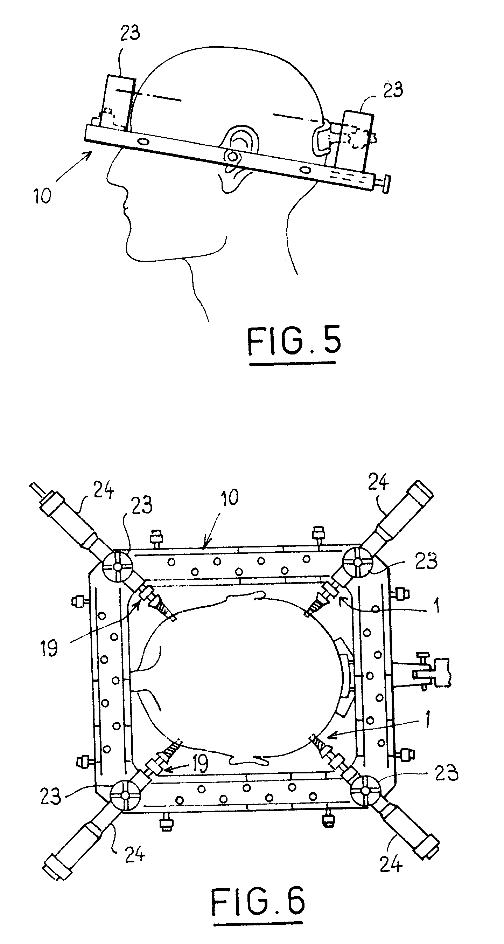

La vis à os percutanée 1 représentée aux dessins est destinée à recevoir un support tel que notamment un cadre 10 de stéréotaxie (Fig.5 et 6). Elle est susceptible de recevoir d'autres applications, par exemple pour la chirurgie maxillo-faciale ou toute chirurgie d'os sous cutané. D'une manière générale, cette vis à os est agencée pour pouvoir refouler toute muqueuse ou aponévrose derrière un os, sans l'endommager.

La vis 1, d'axe XX, comporte une extrémité formée par un téton 2 présentant une surface plane 3 transversale à l'axe XX et une surface latérale cylindrique 4, raccordée à la surface plane 3 par un congé annulaire arrondi 5. Cette vis comprend également une partie filetée 6 munie de filets 7, contigüe au téton 2 et dans laquelle est ménagée une zone autotaraudeuse 8. Cette zone autotaraudeuse est constituée d'au moins une goujure formée par un plat 8, par exemple deux comme dans l'exemple représenté, délimitant une interruption des filets 7 bordée par des arêtes vives 8a, ces plats 8 se terminant à la base du téton 2 ou au voisinage de celui-ci. La vis 1 comprend par ailleurs une partie lisse cylindrique 9, agencée entre sa partie filetée 7 et une tête 11 adaptée pour recevoir un outil de manoeuvre non représenté (tournevis). La partie lisse cylindrique 9 est raccordée à la tête 11 par une zone annulaire plane 12, inclinée d'un angle approprié sur l'axe XX et sur la surface de la partie lisse 9. La zone annulaire 12 est raccordée à la surface transversale 13 de la tête 11 par une partie cylindrique 14 concentrique à l'axe XX.

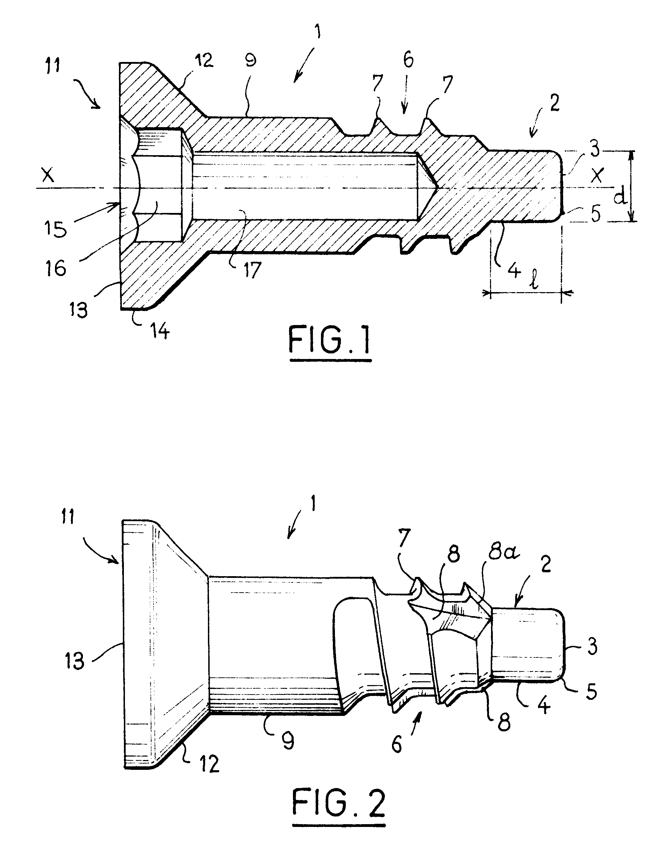

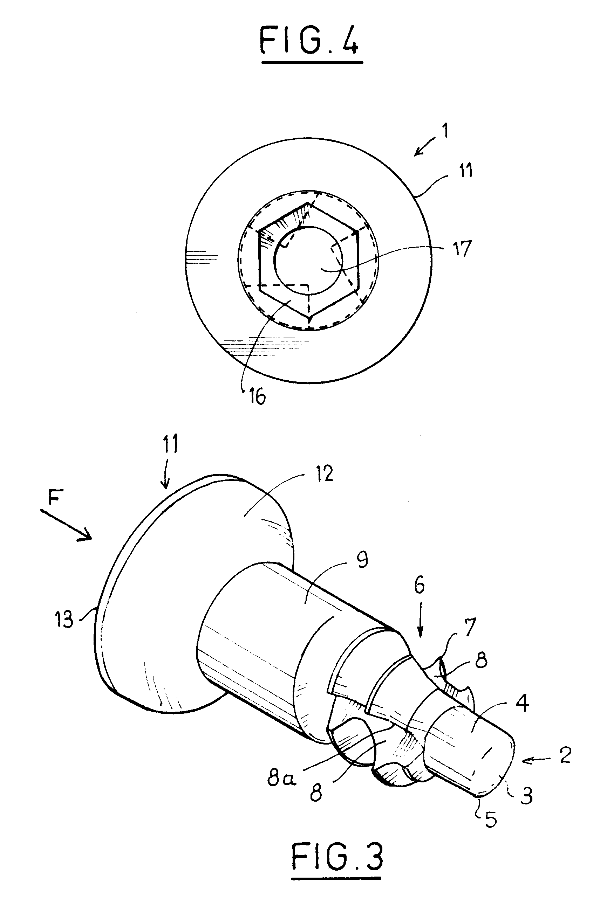

La longueur (1) du téton 2 est au moins égale à son diamètre (d) (sensiblement supérieure à d aux Fig.1 à 3).

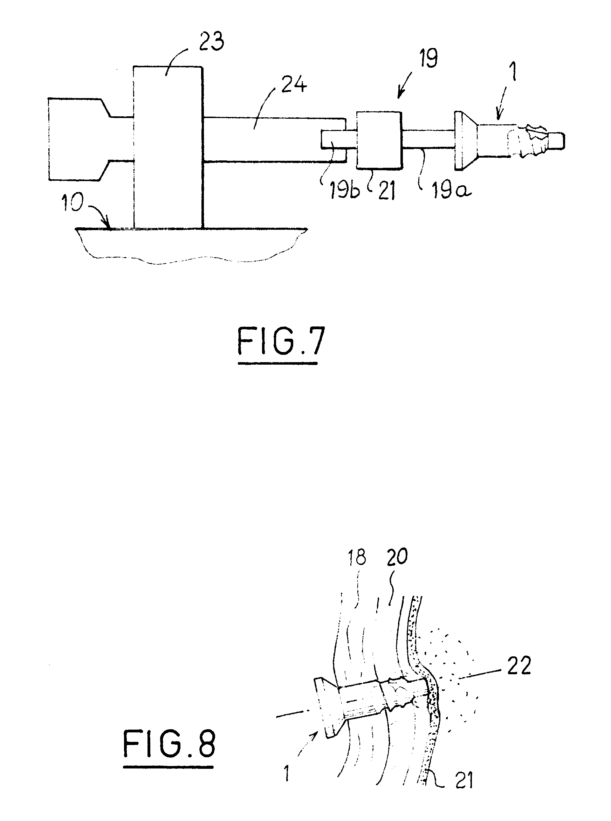

Dans la vis 1 est ménagé un puits axial 15 s'étendant à partir de la tête 11, constitué d'un logement 16 limité par la surface 13 et profilé pour recevoir un outil de vissage, et d'un alésage axial 17 qui prolonge le logement 16 en direction du téton terminal 2. L'alésage 17 est adapté pour recevoir un organe de liaison avec une pièce devant être supportée par des vis 1 ancrées sur le patient, tel que la pièce 19 d'interface avec le cadre 10 de stéréotaxie (Fig.6 et 7).

Dans le cas de l'utilisation de la vis 1 selon l'invention comme support d'un cadre 10 de stéréotaxie, le neurochirurgien ou le médecin, qui procède à la pose des vis 1 nécessaires, les ancre successivement dans la paroi osseuse 20 suivant une inclinaison imposée par le cadre 10 sur la surface de la peau 18 (voir Fig.8) et de l'os crânien 19. Au cours du vissage, la partie autotaraudeuse 8 alèse et taraude le trou de passage nécessaire dans la boîte crânienne 20, puis le téton arrondi 2, après avoir traversé l'os 20, repousse légèrement la dure-mère 21. Cette dernière prend localement la position représentée à la Fig.8 sans être endommagée, et par conséquent sans que l'introduction de la vis 1 ne risque de provoquer une lésion des organes sous-jacents 22. L'alésage de la boîte crânienne 20 par la partie autotaraudeuse 8 et la zone filetée 6 sont susceptibles de provoquer l'apparition d'esquilles d'os, qui ne doivent pas blesser la dure-mère 21. Ce résultat est rendu possible par la forme arrondie du téton terminal 2, qui repousse doucement la dure-mère 21 sans l'agresser et écarte les esquilles sous sa poussée.

De leur côté, les parties lisses 9 et 12, en contact avec la peau 18, ne sont pas susceptibles d'endommager celles-ci avec laquelle elles restent en contact dans leur position définitive, telle que représentée à la Fig.7 dans laquelle la tête 11 fait saillie à l'extérieur de la peau 18.

Le choix de l'agencement des parties lisses 9 et 12 permet, compte tenu de l'inclinaison des vis 1 imposée par le cadre 10, de ne pas endommager la peau du crâne 18 et d'assurer une bonne assise sur la boîte crânienne 20.

Cet ancrage solide permet donc un repositionnement aisé d'un cadre tel que 10, en vue des investigations prévues.

Les organes de liaison entre le cadre 10 et les vis 1 comprennent, dans l'exemple illustré aux Fig.6 et 7, une pièce d'interface 19 munie d'un disque central 21 de part et d'autre duquel font saillie axialement deux parties, antérieure 19a et postérieure 19b. Une pièce d'interface telle que 19 permet le montage d'un cadre 10 sur les vis 1 par l'intermédiaire de quatre piliers d'angle 23 fixés au cadre et qui portent chacun un canon transversal 24 percé de part en part d'un trou axial de réception de la partie postérieure 19a.

Pour mettre en place une vis 1 dans le crâne 20 du patient, le chirurgien introduit un tournevis axialement dans le canon 24 placé sur son pilier-support 23, visse, enlève le tournevis, et pose la pièce d'interface 19 entre la vis 1 et le canon 24 en y introduisant respectivement les parties 19a et 19b.

Après avoir procédé aux examens et investigations prévus, le neuro-chirurgien enlève le cadre 10 et les organes de liaison 19, les vis 1 restant ancrées en place jusqu'au prochain examen.

Avantageusement les vis percutanées 1 sont réalisées en titane, ce qui permet de ne pas déformer les images radiologiques ou IRM (Artefact).

Dans le cadre de son utilisation comme support de système stéréotaxique tel que 10, la vis 1 selon l'invention présente les avantages mentionnés ci-dessus, particulièrement intéressants pour les diverses applications de la stéréotaxie : diagnostic des lésions cérébrales, leur localisation pour leur irradiation ultérieure multifaisceaux, traitement de lésions bénignes, pose de stimulateurs dans le cerveau, électrodes pour repérer d'où viennent des décharges électriques lors de crises d'épilepsie etc.

L'invention n'est pas limitée au mode de réalisation décrit et peut comporter des variantes d'exécution. Ainsi le nombre des goujures autotaraudeuses 8 peut varier : elles peuvent notamment être avantageusement au nombre de trois, séparées angulairement par 120 degrés environ.

Par ailleurs, la forme arrondie du téton 2 peut largement varier : elle peut ainsi notamment être sphérique ou conique à sommet arrondi. La vis illustrée aux dessins peut être modifiée en remplaçant la surface latérale cylindrique 4 par une surface conique ou sphérique. Cette vis peut également être dépourvue de zone autotaraudeuse.

## Revendications

1. Vis à os percutanée (1), destinée à supporter notamment un cadre (10) de stéréotaxie permettant des investigations dans une boîte crânienne, caractérisée en ce qu'elle comporte une extrémité formée par un téton arrondi (2) et émoussé, et en ce que dans sa partie filetée (6), contigüe au téton (2), est ménagée une zone autotaraudeuse constituée d'au moins une goujure formée par un plat (8) bordé par des arêtes vives (8a), et qui se termine au voisinage de la base du téton.

2. Vis selon la revendication 1, caractérisée en ce que le téton (2) présente une surface transversale plane (3) et une surface latérale cylindrique (4) conique ou sphérique, raccordée à la surface plane par un congé annulaire arrondi (5).

3. Vis selon la revendication 2, caractérisé en ce que la partie filetée (6) présente trois zones autotaraudeuses (8) séparées angulairement de 120 degrés environ.

4. Vis selon la revendication 3, caractérisée en ce qu'elle comprend une partie lisse, cylindrique (9), agencée entre sa partie filetée (6) et une tête (11) adaptée pour recevoir un outil de manoeuvre.

5. Vis selon la revendication 4, caractérisée en ce que la partie lisse cylindrique (9) est raccordée à la tête de la vis (1) par une zone annulaire (12), inclinée sur l'axe (XX) de la vis et sur la surface de la partie lisse.

6. Vis selon la revendication 5, caractérisée en ce qu'elle comporte un puits axial (15) s'étendant à partir de sa tête (11), constitué d'un logement (16) profilé pour recevoir un outil de vissage, et d'un alésage axial (17) qui prolonge ce logement en direction du téton terminal (2), cet alésage étant adapté pour recevoir un organe (18) de liaison avec un dispositif tel qu'un cadre (10) de stéréotaxie.

7. Vis selon l'une des revendciations 1 à 6, caractérisée en ce que la longueur (1) du téton (2) est au moins égale à son diamètre (d).
